# EUROPEAN PATENT APPLICATION

(11) **EP 2 353 604 A1**
(43) Date of publication of application: **10.08.2011**
(21) Application number: 10152495.7
(22) Date of filing: 03.02.2010
(51) Int. Cl.: A61K 36/748, A61K 36/41, A61P 35/00

(54) **Pharmaceutical composition and health food comprising rhodiola sachalinensis and oldenlandia diffusa for preventing and treating cancer**

(71) Applicant: Nam, Jong Hyun, Songpa-gu, Seoul 138-110 (KR)
(72) Inventor: Nam, Jong Hyun, Songpa-gu, Seoul 138-110 (KR)
(74) Representative: von Kreisler Selting Werner

(57) **Abstract**

A pharmaceutical composition for treating and preventing cancer, and health food for ameliorating and preventing cancer containing the pharmaceutical composition are provided. The pharmaceutical composition includes *rhodiola sachalinensis, oldenlandia diffusa,* and *cistanchis herba* to increase the expression of a cancer suppressor gene and thus treat and prevent cancer by suppressing the growth of cancer cells while not affecting normal cells.

## Description

### BACKGROUND

### 1. Field

Example embodiments relate to a pharmaceutical composition for preventing and treating cancer and a health food containing the same for amelioration and treatment of cancer, and more particularly, to a pharmaceutical composition for preventing and treating cancer with minimal side effects, which prevents proliferation of cancer cells through promoting expression of cancer suppressor genes using *Rhodiola sachalinesis* and *Oldenlandia diffusa,* and a health food including the same as an effective ingredient for amelioration and prevention of cancer.

### 2. Description of the Related Art

Cancer occurs as abnormal tissue masses in a living host organism that receive nutrition from the host to excessively proliferate independent of the host and destroy the host organism. Organs of the human body are formed of large numbers of cells. When normal cells of the human body change into abnormal cells and the abnormal cells divide and proliferate unchecked, cancer occurs. Although genetics factors are deeply involved in cancer contraction, environmental factors also greatly affect whether an individual contracts cancer. The occurrence of the cancer is particularly prevalent in developed countries. It has been reported that cancer is caused by increased use of pesticides, insecticides, and the like and thus residual amounts of such substances in food, increased consumption of processed food containing addictives such as food preservatives and coloring agents, increased pollution of water, soil, and air, stress of modem living, reduced activity, obesity caused by a rich dietary habits, and the like. In recent years, it has been also noted that cancer is caused when the cell signaling system for normal cells malfunctions, when a cancer gene is activated, or when a cancer suppressor gene malfunctions.

Various cancer treatment methods exist such as surgical treatment, chemotherapy, and radiation treatment. Surgical treatment method is effective for removing cancer in the early stages, but has the drawbacks of sometimes having to remove organs, which causes side effects, and being uncertain in containing the spread of cancer to other organs. Radiation treatment is advantageous in effectively treating cancer occurring in a specific organ but has the drawbacks of patients exposed to the risk of other cancers due to the radiation, being unable to prevent the spread of cancer to other organs, and patients suffering pain during treatment. Chemotherapy is generally performed using anti-cancer medicine, but the toxicity of anti-cancer medicine is known to act not only on cancerous cells but also normal cells of a patient, causing side effects. Therefore, new anti-cancer drugs are being developed to have higher selectivity of cancer cells and minimal toxicity.

It is known that naturally-occurring ingredients have less toxicity and side effects, which has recently fueled vigorous research efforts to develop various medicinal or drink preparations by extracting anti-cancer ingredients from natural materials or processing natural materials. However, the toxicity of some of these new drink preparations containing natural medicinal ingredients may act not only on cancer cells but healthy cells as well, causing loss of hair and other side effects in patients. In addition, since the new natural medicinal preparations tend to be expensive, their high cost prohibits their wide distribution.

### SUMMARY

Embodiments are directed to a pharmaceutical composition for preventing and treating cancer and health food containing the pharmaceutical composition as an effective ingredient, which substantially overcome one or more of the problems due to the limitations and disadvantages of the related art.

It is therefore a feature of an embodiment to provide a pharmaceutical composition for preventing and treating cancer and health food containing the pharmaceutical composition as an effective ingredient, which can suppress activity of cancer cells by acting only on cancer cells without any side effects on normal cells and increasing expression of the anti-cancer gene.

At least one of the above and other features and advantages may be realized by providing a composition for treating and preventing cancer, including *rhodiola sachalinensis* and *oldenlandia diffusa.*

The *rhodiola sachalinensis* may be 10-70 parts by weight for the composition of 100 parts by weight.

The *oldenlandia diffusa* may be 20-80 parts by weight for the composition of 100 parts by weight.

The composition may further include *cistanche deserticola.*

The *cistanche deserticola* may 10-40 parts by weight for the composition of 100 parts by weight.

The composition may further include one selected from the group consisting of *Torilis japonica, Cuscuta japonica, Polygala tenuifolia,* and a mixture thereof.

*Torilis japonica, Cuscuta japonica Chois, Polygala tenuifolia,* or a mixture thereof may be 5-15 parts by weight for the composition of 100 parts by weight.

The composition may increase expression of a cancer suppressor gene.

The cancer may be one of pancreatic cancer, liver cancer, stomach cancer, colon cancer, uterine cancer, breast cancer, lung cancer, and prostate cancer.

At least one of the above and other features and advantages may be realized by providing a health food for ameliorating and preventing cancer, comprising the above composition for treating and preventing the cancer as an effective ingredient.

The health food may be a health beverage.

The health food may be a natural tea.

The composition may be 0.001 to 30 parts by weight for the health food of 100 parts by weight.

### BRIEF DESCRIPTION OF THE DRAWINGS

The above and other features and advantages will become more apparent to those of ordinary skill in the art by describing in detail exemplary embodiments with reference to the attached drawings, in which:

FIG. 1 is a mimetic diagram illustrating a cell death mechanism and cell cycle regulatory mechanism by activation of p53;

FIG. 2 is a graph illustrating comparison results of the viability of cells that were treated with health beverages of Embodiments 3 to 6 and cultivated for 24 hours, based on 100% cell viability before the cells were not treated with the health beverages;

FIG. 3 illustrates microscopic views of PANC1 cells which were treated with samples of Embodiments 3 to 6 and cultivated for 24 hours;

FIG. 4 illustrates a comparison of p21 protein expression in human cancer cell lines when the cell lines are and are not treated with samples of embodiments;

FIG. 5 illustrates a comparison of Bax protein expressoin in human cancer cell lines when the cell lines are and are not treated with samples of embodiments;

FIG. 6 is a view illustrating the transplantation of a human cancer cell line to a nude mouse;

FIG. 7 is a graph illustrating the effect that a health beverage containing a composition of an embodiment has on the amount of food intake by a mouse model to which cancer cells have been transplanted;

FIG. 8 is a graph illustrating the effect that a health beverage containing a composition of an embodiment has on the amount of beverage intake by a mouse model to which cancer cells have been transplanted;

FIG. 9 illustrates sacrificed nude mice models to which cancer cells were transplanted and pharmaceutical compositions of embodiments were fed for two weeks;

FIG. 10 illustrates weights of tumors extracted from nude mice models to which cancer cells were transplanted;

FIG. 11 illustrates weights of pancreatic carcinoma tissue extracted from nude mice models to which cancer cells were transplanted;

FIG. 12 illustrates weights of prostate cancer tissue extracted from nude mice models to which cancer cells were transplanted; and

FIG. 13 are graphs illustrating the effect that pharmaceutical compositions of embodiment have on p53 and p21 protein expression after extracting pancreatic carcinoma tissue from nude mice models to which cancer cells were transplanted.

### DETAILED DESCRIPTION

Example embodiments will now be described more fully hereinafter with reference to the accompanying drawings; however, they may be embodied in different forms and should not be construed as limited to the embodiments set forth herein. Rather, these embodiments are provided so that this disclosure will be thorough and complete, and will fully convey the scope of the invention to those skilled in the art.

A pharmaceutical composition for preventing and treating cancer of an embodiment includes *rhodiola sachalinensis* and *oldenlandia diffusa.*

The pharmaceutical composition of this embodiment increases expression of a cancer-suppressor gene and suppresses proliferation of cancer cells. Therefore, the pharmaceutical composition is effective in preventing and treating the cancer. In addition, since the pharmaceutical composition of this embodiment is formed of natural materials, it does not affect normal cells while suppressing the activity of the cancer cells and thus there are fewer side effects from the pharmaceutical composition.

The *rhodiola sachalinensis* is also called Rhodiola sachalinensis A. Bor that is a perennial herb that grows wild high in the mountains at 1800-2300m above sea level. Biologically-active components of the *rhodiola sachalinensis* increase the expression of cancer suppressor genes such as p53, p21, and Bax to suppress proliferation of cancer cells.

The suppression and activation of specific genes causes cancer generation and development. An oncogene promotes the growth of cancer and a cancer-suppressor gene suppresses the growth of cancer. The p53, p21, and Bax are representative cancer suppressor genes.

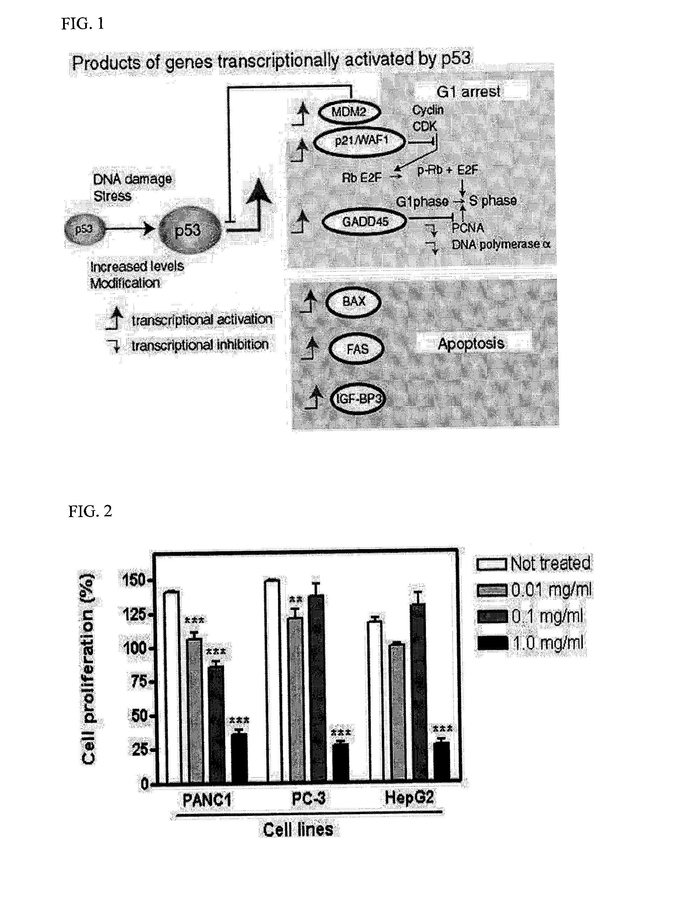

FIG. 1 is a mimetic diagram illustrating a cell death mechanism and cell cycle regulatory mechanism by activation of p53. The p53 is the most well-known cancer-suppressor gene, which functions to protect the cells such that the cells are not changed into malignant cells by regulating the cell cycle and inducing cell death. The variation and loss of the p53 gene is one of most common genetic changes among human cancers. When cells experience DNA damage, the expression of p53 steeply increases and thus the expression of the p21 that is a subordinate gene of p53 is induced, thereby stopping the cell cycle at a G1-phase. CD95/FAS, Bax, Noxa, Capase-1, and the like in addition to the p21 are known as the subordinate genes of p53, which induce cell death and are regulated by p53. In addition, the p53 activates GADD45 involved in DNA excision repair to promote the damaged DNA excision repair, thereby maintaining the stability of the gene.

The p21 is a subordinate gene of p53 and suppresses the cell cycle progress at the G1-phase. The cell cycle is divided into G1, S, and G2, and M-phases. It is very closely related to phosphorylation of retinoblastoma (Rb) whether the cell cycle stops at the G1-phase or proceeds from the G1-phase to the S-phase to proliferate the cells. Non-phosphorylated pRb suppresses the expression of the the genes required for the S-phase through the suppression the function of the E2F by binding with the E2F. Thus, the cell cycle stops at the G1-phase. However, when pRb is phosphorylated by compound of cyclin and cyclin-dependent kinase (CDK) complex, the pRb is not coupled to the E2F anymore and thus the cell cycle proceeds by the activation of the transcription of the genes required for the S-phase due to the activation of the isolated E2F. In this process, the p21 suppresses the activation of the cyclin-CDK complex to suppress the phosphorylation of the pRb. Therefore, the E2F in an inactivated state keeps the coupling to the pRb and thus the cell cycle stops at the G1-phase. As a result, the proliferation of the oncogene can be suppressed.

The Bax protein is the most important factor controlling cell death. When activated p53 expressed protein exists in protoplasm, the activation of the Bax protein increases. The increase of the activation of the Bax protein forms holes in the cell wall of mitochondria existing in the cells. The mitochondria are a small part producing ATP required for existing and activation of the cells through the electron transport system. Therefore, it becomes difficult for the cells to survive by the holes formed in the mitochondrion wall. Therefore, when the expression of the Bax protein in the tumor cells increases, cell death is promoted to remove the tumor cells.

The rhodiola sachalinensis may be thoroughly used or only a specific portion (e.g., leaf, root, or stem) may be used.

The *rhodiola sachalinensis* may be added in the form of an extract. The extract may be lyophilized or dried by hot wind in the form of powders containing moisture content of 5-20%. The *rhodiola sachalinensis* may be added as-is without being extracted after being dried and powdered. The extraction may be performed by any extracting methods. Especially, the organic solvent extraction method may be used. At this point, water, ethanol, or a mixture of the water and ethanol, not harmful to human, may be used as the organic solvent. At this point, 10-70% ethanol may be used. The extraction is performed for 5-20 hours at a temperature of 25-100 °C.

For a composition of 100 parts by weight, the *rhodiola sachalinensis* may be added by 10-70 parts by weight. When the *rhodiola sachalinensis* is added to the composition by less than 10 parts by weight, it is not effective to prevent and treat cancer. When the *rhodiola sachalinensis* is added to the composition by greater than 70 parts by weight and the composition is added to health food, the sense of taste may be deteriorated since the typical taste and flavor of the *rhodiola sachalinensis* are rich and the enhancement of the effect is imperceptible compared to the amount of content.

The *oldenlandia diffusa* has an effect in suppressing the growth of the oncogene. When the *oldenlandia diffusa* is used together with the *rhodiola sachalinensis,* it can be expected that the cancer treatment and prevention effects can be further enhanced. In addition, even when *the oldenlandia diffusa* is taken for a long time, it has no side effects.

The *oldenlandia diffusa* may be added in the form of an extract. The extract may be dried in the form of powders and added. The *oldenlandia diffusa* may be added as-is without being extracted after being dried and powdered. The extraction may be performed by any extracting methods. Especially, the organic solvent extraction method may be used. At this point, water, 10-70% ethanol, or a mixture of the water and ethanol may be used as the organic solvent.

For a composition of 100 parts by weight, the *oldenlandia diffusa* may be added by 20-80 parts by weight. When the *oldenlandia diffusa* is added to the composition by less than 20 parts by weight, it is not effective to generate the cancer-suppressing gene and prevent and treat cancer. When the *oldenlandia diffusa* is added to the composition by greater than 80 parts by weight, the increase of the anti-cancer effect is insignificant as compared with the increased amount of the *oldenlandia diffusa* and it induces weak diarrhea.

The pharmaceutical composition for treating and preventing cancer according to this embodiment may further include *cistanche deserticola.*

When the *cistanche deserticola* is used together with the *rhodiola sachalinensis* and *oldenlandia diffusa,* the effect in suppressing the growth of the cancer cells can be further enhanced.

The *cistanche deserticola* may be added in the form of an extract. The extract may be dried in the form of powders and added. The *cistanche deserticola* may be added as-is without being extracted after being dried and powdered. The extraction may be performed by any extracting methods. Especially, the organic solvent extraction method may be used. At this point, water, 10-70% ethanol, or a mixture of the water and ethanol may be used as the organic solvent.

For a composition of 100 parts by weight, the *cistanche deserticola* may be added by 10-40 parts by weight. When the *cistanche deserticola* is added to the composition by less than 10 parts by weight, it is not effective to enhance the effect in suppressing the growth of the cancer cells. When the cistanche deserticola is added to the composition by greater than 40 parts by weight and the composition is added to health food, the sense of taste may be deteriorated due to the typical flavor of the *cistanche deserticola.*

The pharmaceutical composition for treating and preventing the cancer according to this embodiment may further include one selected from the group consisting of *Torilis japonica, Cuscuta japonica Chois, Polygala tenuifolia ,* and a mixture thereof to further enhance the cancer treatment and prevention effects.

The *Torilis japonica* keeps the liver warm and is good for the stamina. The *Torilis japonica* is effective for anti-inflammation and thus it is also used to treat eczema and dermatitis caused by allergy. In this embodiment, the Torilis japonica functions to suppress generation of a blood vessel. The spreading of the cancer occurs through the generation of the blood vessel. Therefore, when the *Torilis japonica* is added to the composition, the cancer spreading can be suppressed.

It is known that the *Cuscuta japonica Chois* is effective in suppressing tumors and is good for nutritious vigorousness. The antibody can be formed by the *Cuscuta japonica Chois* early, the anti-cancer effects can be further enhanced.

The *Polygala tenuifolia* is a root of *Polygala tenuifolia Willd* that is a member of the Polygala tenuifolia family. The *Polygala tenuifolia* is effective in relaxing the mind and further enhances increasing effects of the cancer-suppressor gene expression.

The *Torilis japonica, Cuscuta japonica Chois,* and/or *Polygala tenuifolia* may be added in the form of an extract. The extract may be lyophilized or dried by hot wind in the form of powders. The *Torilis japonica , Cuscuta japonica Chois,* and/or *Polygala tenuifolia* may be added as-is without being extracted after being dried and powdered.

For a composition of 100 parts by weight, *Torilis japonica, Cuscuta japonica Chois, Polygala tenuifolia* or mixture thereof may be added by 5-15 parts by weight. When *Torilis japonica , Cuscuta japonica Chois, Polygala tenuifolia* or mixture thereof is added to the composition by less than 5 parts by weight, it is not effective to enhance cancer treatment and prevention effects. When *Torilis japonica, Cuscuta japonica Chois, Polygala tenuifolia* or mixture thereof is added to the composition by greater than 15 parts by weight, the enhancement of anti-cancer effect is insignificant.

The pharmaceutical composition for treating and preventing cancer according to this embodiment may, as occasion demands, selectively include one or more selected from the group consisting of licorice, honey, *Angelica gigas, Maximowiczia typica,* jujubes, ginseng, red ginseng, ginger, and the like.

These components are added to harmonize the effects of other medicines and to improve the sense of taste.

The pharmaceutical composition for treating and preventing the cancer according to this embodiment may be effective for pancreatic cancer, prostate cancer, stomach cancer, colon cancer, uterine cancer, breast cancer, lung cancer, and liver cancer, particularly, for pancreatic cancer, prostate cancer, and liver cancer.

The pharmaceutical composition can be more effectively used for the pancreatic cancer since the anti-cancer effect is achieved even with low concentration. No effective treatment method has been developed for the pancreatic cancer though a variety of treatment methods such as chemical treatment and radiation treatment have been used.

The pharmaceutical composition for treating and preventing the cancer according to this embodiment may be prepared in the form of an extract by individually extracting the above natural medicine materials or in the form of powders. Alternatively, after washing the natural medicine materials, they are chopped and mixed with each other and then the composition is extracted from the mixture. The extraction may be performed by any extracting methods. Especially, the organic solvent extraction method may be used. At this point, drinkable water, ethanol, or a mixture of the water and 10-70% ethanol may be used as the organic solvent.

The extract may be concentrated and lyophilized or dried by hot wind in the form of powders.

The following will describe the extracting and powdering processes in more detail.

The natural medicine materials are washed and dipped in 30-70% ethanol, preferably 50% ethanol for 30 minutes to 4 hours at a temperature of 60°C or less. After this, extraction is performed for 1-10 hours at a temperature of 80-100°C. After the extraction, the extract is centrifugal-separated for 5-30 minutes at 5,000-10,000 rpm and then the supernatant is removed by a filter. After this, the separated material is concentrated at a reduced pressure to 75°Brix at a temperature of 30-70°C. The concentration is dried by hot wind to contain moisture of 10% or lyophilized at a temperature of-40°C or less, after which the concentration is powered.

The pharmaceutical composition for treating and preventing the cancer according to this embodiment may be mixed with carriers that are pharmaceutically allowable. That is, forming agents, disintegrants, sweeteners, lubricants, flavoring agents, and the like may be added to the composition. In addition, the composition may be provided in the form of a tablet, a capsule, an agent, a granule, suspension, an emulsifying agent, syrup, and the like through well-known methods in the art.

The pharmaceutical composition for treating and preventing cancer according to this embodiment may be orally or non-orally administered. The pharmaceutical composition may be administered as an effective component by 0.01-10g per 1kg weight once or several times. An amount of medication or dosage may be properly adjusted depending on weight, age, sex, and physical condition of the patient, a medication time and method, an excretion rate, and a degree of symptoms.

A health food for preventing and improving cancer according to one embodiment may include the above-described pharmaceutical composition for treating and preventing the cancer. The health food may be made in the form of health beverage. For example, the health food containing the pharmaceutical composition for treating and preventing the cancer as an effective component may be made in the form of a natural tea.

The health food or health beverage containing the pharmaceutical composition for treating and preventing the cancer as an effective component is effective in improving and preventing the cancer and has no side effects. Particularly, the health beverage is cheap and is easy for a patient to take.

For the health food or beverage of 100 parts by weight, the composition is added to the health food or beverage by 0.001-30 parts by weight. When the composition is added by less than 0.001 parts by weight, the cancer improvement and prevention effect is not significant. When the composition is added by greater than 30 parts by weight, the taste of the food or beverage is deteriorated due to the typical taste and flavor of the composition.

Hereinafter, the present invention will be described in more detail using embodiments and comparative examples, which will not limit the scope of the invention.

<Embodiment 1> Preparation of Extract of Composition Effective in Treating and Preventing Cancer

30g of *rhodiola sachalinensis,* 20g of *oldenlandia diffusa,* 20g of *cistanche deserticola,* and 10g of *Polygala tenuifolia* were washed by water and dried. Then, they were chopped and dipped in 50% ethanol for 1 hour at a temperature of 50°C, after which extraction was performed using 50% ethanol for 10 hours and 10g of honey was added to the extract. The extract contains a moisture level of 25%.

<Embodiment 2> Preparation of Power of Composition Effective in Treating and Preventing Cancer

The extract attained in Embodiment 1 was centrifugal-separated for 15 minutes at 7500 rpm, and supernatant was removed by a filter, after which the separated extract was concentrated under atmospheric pressure of 70 at a temperature of 60°C. The concentration was dried by hot wind for 30 minutes at a temperature of 70°C so that the concentration contains a moisture level of 5%.

<Embodiments 3-6> Preparation of Health Beverage

The extract attained in Embodiment 1 and the powder attained in Embodiment 2 were mixed with each other at a compositing ratio shown in the following Table 1 and the mixture was diluted with water so that the total volume became 140 ml to prepare the health beverage.

**[Table 1]**

| | Extract (g) Of Embodiment 1 | Powder (g) of Embodiment 2 (weight when converted into extract) | Amount of Extract/100g Beverage |
|---|---|---|---|
| Embodiment 3 | 10 | 0.5 (0.633) | 7.6 |
| Embodiment 4 | 10 | 1(1.267) | 8.0 |
| Embodiment 5 | 20 | 2 (2.533) | 16.0 |
| Embodiment 6 | 30 | 4 (5.067) | 25.0 |

<Test Example 1> Identification of Cell Proliferation Suppressing Effect

The cell line proliferation suppressing effects of the health beverages of the embodiments were measured using MTT assay. A PANC1 cell that is a human pancreatic carcinoma cell line, a HepG2 cell that is a human hepatocellular carcinoma cell line, and a PC-3 cell that is a human prostate cancer cell line were line-divided to 1.5 X 10⁴ at a 96 well microplate and cultivated for 24 hours under a condition of a temperature of 37°C and 5% CO₂. When the cells were grown up to 80% in the well, the health beverages of Embodiments 3-6 were processed to have concentrations of 0.01 mg/ml, 0.1 mg/ml, and 1 mg/ml and cultivated for 24 hours.

In order to measure the cell growth suppressing effect, a 5mg/mℓ 3-(4,5-dimethylthiazol-2.5-dimethyltetrazolium bromide (MTT; sigma, USA) solution was added to each well by 20 *µℓ* and cultivated for 4 hours at a temperature of 37°C . After this, media were removed from the microplate and 200 *µℓ* of dimethyl sulfoxide (DMSO) solution was added to each well. Color reaction of formazan and DMSO solution formed by the MTT solution was measured by measuring extinction at 540 nm. The results are shown in the following Table 2 and FIG. 2.

**[Table 2]**

| Concentration (mg/ml) | Embodiment | Cell Proliferation (%) | | |
|---|---|---|---|---|
| | | Panc1 | PC-3 | HepG2 |
| 0.01 | Embodiment 3 | 77.08±1.597 | 90.80±0.812 | 94.50±3.254 |
| | Embodiment 4 | 81.39±0.928 | 82.97+1.860 | 88.81±1.763 |
| | Embodiment 5 | 80.38±1.179 | 84.79± 1.207 | 80.86±1.376 |
| | Embodiment 6 | 65.50±1.855 | 71.777±1.876 | 83.33±1.967 |
| | Mean Value | 76.09±3.647 | 82.58±3.973 | 86.88±3.036 |
| 0.1 | Embodiment 3 | 68.79±1.037 | 102.92±1.821 | 113.88±3.937 |
| | Embodiment 4 | 62.46±0.719 | 101.00±1.453 | 118.95±4.550 |
| | Embodiment 5 | 54.68±0.652 | 79.20±0.853 | 126.03±4.237 |
| | Embodiment 6 | 58.07+1.197 | 85.03+1.162 | 108.51±3.725 |
| | Mean Value | 61.00±3.046 | 92.04±5.864 | 116.84±3.731 |
| 1.0 | Embodiment 3 | 22.49±0.849 | 14.01±0.287 | 18.01±0.113 |
| | Embodiment 4 | 20.84+0.437 | 16.07±0.376 | 21.12±1.223 |
| | Embodiment 5 | 24.79±0.324 | 17.81±0.405 | 22.77±0.321 |
| | Embodiment 6 | 32.11±0.211 | 23.63±0.368 | 30.06±1.070 |
| | Mean Value | 25.06±2.486 | 17.88±2.068 | 23.13±2.680 |

| | | | | |
|---|---|---|---|---|
| The data shown are means±SEM. | | | | |

The results are expressed as percentage viability with non-treated cells being set at 100% viability.

Table 2 shows results of comparison of viabilities of cells that are treated with the health foods of the embodiments with reference to viability (100%) of the cells cultivated for 24 hours at media that are not treated with the health beverages of Embodiments 3-6. As shown in Table 2, all of the health beverages of Embodiments 3-6 suppress the proliferation of the PANC1 cell. For the PC-3 cell, the cell viability was reduced on when the cell was processed by the beverages of Embodiment 5 and 6 with the concentration of 0.1 mg/ml. For the HepG2 cell, the cell viability was reduced only when the cell was processed with the concentration of 1.0 mg/ml.

FIG. 2 shows results of comparison of a viability of the cell that was not processed by the health beverages of the embodiments and cultivated for 24 hours and a viability of the cell that is processed by the health beverages of the embodiments with reference to a viability (100%) before the cell was processed by the health beverages of Embodiments 3-6. As shown in FIG. 2, when the PANC1 cell, HepG2 cell, and PC-3 cell were not processed by the beverages of the embodiments, the viabilities thereof were respectively 140.5%, 116.6%, and 148.4%. This shows that the cell proliferation increases as compared with a case before the cells are processed. For the PANC1 cell, when it was processed by the health beverages of Embodiments 3-6, the cell viability was reduced as compared with a case before it is processed by the health beverage. In addition, the PANC1 cell tends to depend on the concentration. That is, the proliferation of the PANC1 cell was further suppressed as the concentration increases. For the PC-3 cell and the HepG2 cell, when they are processed by the beverages with concentration of 0.01 mg/ml and 0.1 mg/ml, the cell viability was not reduced, and only when processed by the beverages with concentration of 1 mg/ml was the cell viability reduced.

Therefore, the proliferation of the pancreatic carcinoma cell line can be suppressed even when it was processed by the health beverages of Embodiments 3-6 with the concentration of 0.01 mg/ml. However, for the hepatocellular carcinoma cell line and the prostate cancer cell line, no cell proliferation suppressing effect was attained when they are processed by the health beverages of Embodiments 3-6 with concentration of 0.01 mg/ml and 01 mg/ml.

<Test Example 2> Morphological Observation of Cell

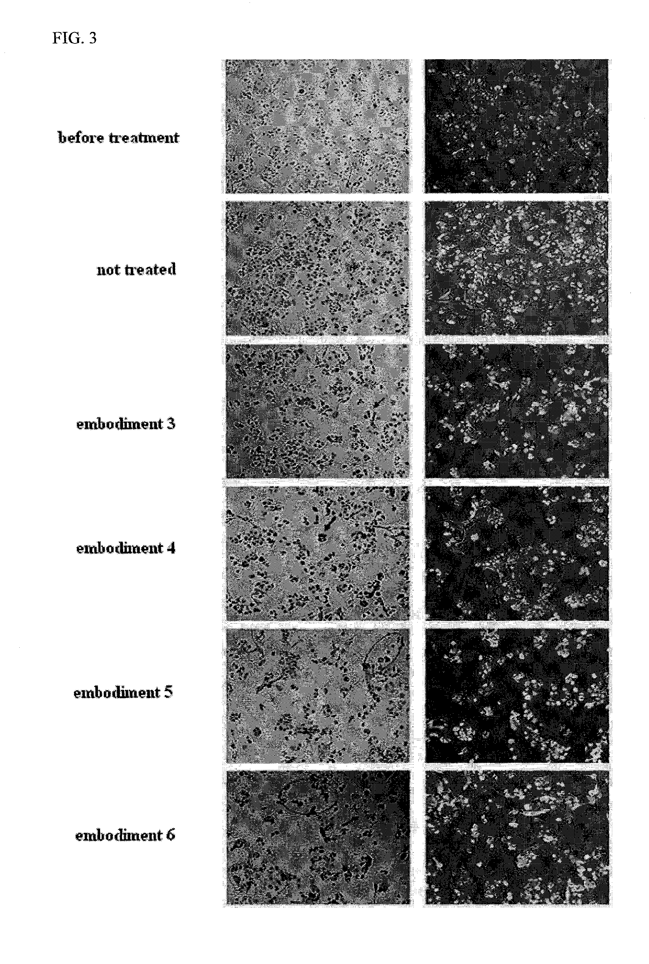

The cell proliferation suppressing effect of the health beverages of Embodiments 3-6 was morphologically observed. The PANC1 cell was line-divided with concentration of 3.0X10⁶ cells/well at 100 mm culture dish and cultivated for 24 hours at a temperature of 37°C under a 5% CO₂ atmosphere. When the cell was grown up to 80% in the culture dish, the health beverages of Embodiments 3 to 6 were processed with concentration of 1 mg/ml and cultivated for 24 hours. The cells processed by the beverages of Embodiments 3-6 were washed two times by PBS buffer and the configuration of the cells were observed at 2X magnification using EVOS digital inverted microscope. The results are shown in FIG. 3.

In FIG. 3, a scattered portion around the cell and a portion where the number of cells is reduced are indicated by an arrow. "Before process" means that the cells are not processed by the health beverages and cultivated for 24 hours. As shown in FIG. 3, portions around the cells that are not processed by the health beverages of Embodiments 3-6 was further grown up and the cancer cells were normally and densely grown up. On the contrary, portions around the cells that are processed by the health beverages of Embodiments 3-6 were scattered and dead. When the cells were processed by the health beverage of Embodiment 6, the number of cells was greatly reduced. This shows that the cancer cell proliferation suppressing effect is more excellent as the concentration of the composition increases.

<Test Example 3> Identification of Effect on Generation of Cancer Suppressing Factors

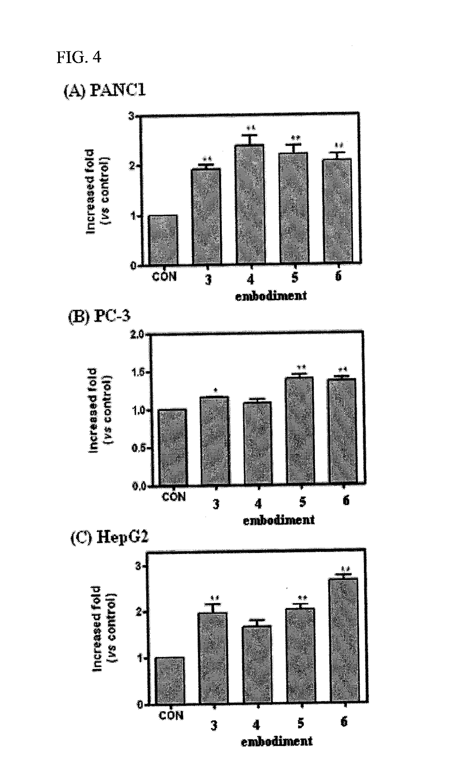

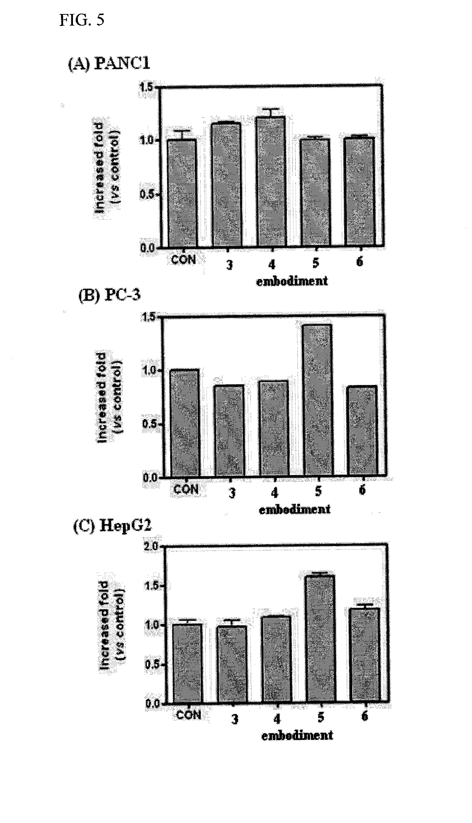

Effects of the health beverages of Embodiments 3-6 containing the pharmaceutical composition of the embodiment on the generation of p21 and Bax that are subordinate genes controlled by p53 in a human cancer cell line were researched using a western blot method. The PANC1 cell, HepG2 cell, and PC-3 cell were line-divided with concentration of 3.0X10⁶ cells/well at 100 mm culture dish and cultivated for 24 hours at a temperature of 37°C under a 5% CO₂ atmosphere. When the cells were grown up to 80% in the culture dish, the health beverages of Embodiments 3 to 6 were processed with concentration of 1 mg/ml and cultivated for 24 hours, after which the cells are recovered. The recovered cells were washed three times by PBS buffer and lightly pipetted after adding lysis buffer of 40-80 *µℓ*. The cells were incubated in ice for 30 minutes and a sonication process was performed for 30 seconds. After this, the cells were centrifugal-separated for 15 minutes at 13,000 rpm and 4°C to separate the protoplasm. An amount of protein of the separated protoplasm was quantified using a BCA reagent. The protein was separated by electrophoresis of each protoplasm of 35 *µg* in 15% SDS-PAGE gel. After this, the separated protein was transferred to nitrocellulose (NC) membrane by a semi-dry machine and the NC membrane was blocked overnight at a temperature of 4°C using a blocking buffer, after which it was washed by 1XTBS-T. Primary antibodies of the p21 and Bax were diluted with IXTBS-T with 1:1200 and reacted overnight at a temperature of 4°C. The NC membrane that went through the primary antibody reaction were washed three times each for 10 minutes using the 1XTBS-T buffer, after which a secondary antibody reaction was performed for two hours at a temperature of 4°C. The secondary antibody for the p21 used horseradish peroxidase (HRP)-conjugated goat anti-rabbit LgG. The secondary antibody for the Bax used horseradish peroxidase (HRP)-conjugated rabbit anti-goat LgG. After the reaction, the antibodies were reacted with a western blotting luminal reagent and developed on X-ray film, after which the protein band was observed. The results are shown in FIGS. 4 and 5 with reference to the p21 and Bax protein generation (1.0) in a control group that was not treated with the health beverages of Embodiments 3-6.

As shown in FIG. 4, in all of the PANC1 cell, PC-3 cell, and HepG2, the generation of the p21 protein was increased as compared with a case where the cells are not treated with the health beverages of Embodiments 3-6. Particularly, in the PANC1 cells treated with the beverage of Embodiment 4, the generation of the p21 was increased by 2.4 times as compared with the case where the PANC1 cells were not treated with the beverage of Embodiment 4. When the PANC 1 cells were treated with the beverages of Embodiments 5 and 6, the generation of the p21 was increased by 2 times (see FIG. 4A). In HepG2 cell, the generation of the p21 protein was increased (see FIG. 4C). In the PC-3 cell, the generation of the p21 protein was increased but less than the PANC1 and HepG2.

Referring to FIG. 5, the Bax protein was not significantly increased even when the cells are processed by the beverages of Embodiments 3-6. However, for the PC-3 cell and HepG2 cell, the generation of the Bax protein was increased when they are treated with the beverage of Embodiment 5.

From the above results, the cancer cell growing suppressing by the composition of the embodiment is more affected by the increase of the generation of the p21 protein than the increase of the generation of the Bax protein.

<Test Example 4> Measuring of Amounts of Food Injection and Drink Injection in Vivo Model

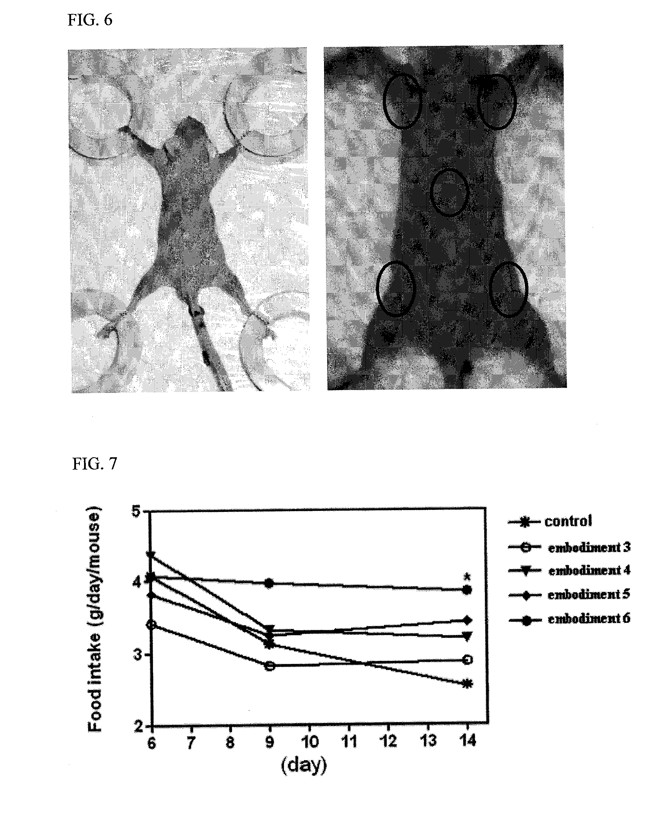

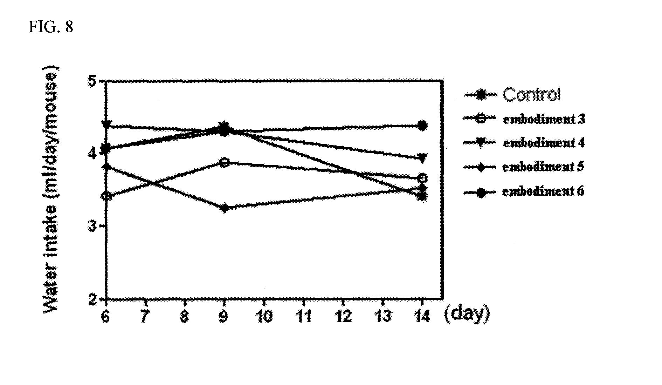

The PANC1 with concentration of 5x10⁵ cells/0.2 ml was inoculated to subcutaneous fat layers of front legs of nude mice. The HepG2 with concentration of 7.55x10⁵ cells/0.2 ml was inoculated to subcutaneous fat layers of backs. The FC-3 with concentration of 5x10⁵ cells/0.2 ml was inoculated to subcutaneous fat layers of rear legs (see FIG. 6). The test was performed for 2 weeks with one control group and 4 experimental groups. Sterilized distilled water was supplied to the control group as a beverage. The health beverages of Embodiments 3-6 are diluted to the sterilized distilled water to have concentration of 1 mg/ml were supplied to the experimental groups as beverages. While supplying the pharmaceutical materials, an amount of food ingestion and an amount of beverage ingestion were recorded as shown in FIGS. 7 and 8.

As shown in FIGS. 7 and 8, the control group has the same amounts of the food and beverage ingestions as the experimental groups until 6 days from the test start day has passed. However, the amounts of the food and beverage ingestions of the control group keep decreasing as the days have further passed. The amounts of the food and beverage ingestions of the experimental groups taking the health beverages of Embodiments 3-6 were increased after decreasing for a while or were not reduced.

<Test Example 5> Effect on Suppressing of Tumor Growth

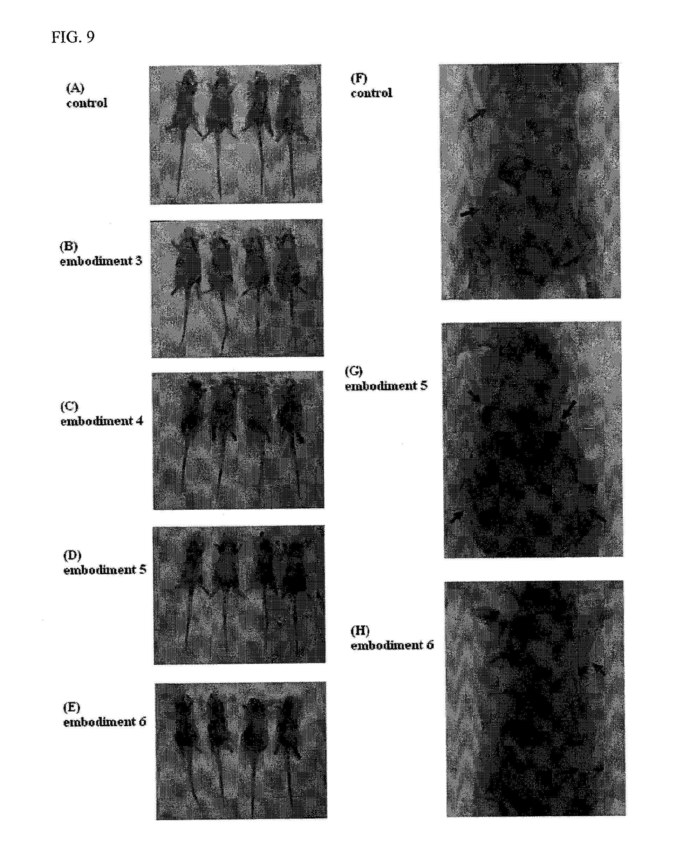

The beverages with concentration of 1mg/ml of Embodiments 3-6 were fed for two weeks to the nude mice to which the cancer cells are transplanted in the same method of Test Example 4, after which the mice were sacrificed, pictures of which are shown in FIG. 9. The tumors are indicated by arrows (see FIG. 9). The human pancreatic carcinoma cell lines were inoculated to the left and right front legs and the human prostate cancer cell line were inoculated to the left and right rear legs. FIG. 9A shows the control group supplied with the sterilized distilled water, FIG. 9F shows the experimental groups, FIG. 9G shows individuals supplied with the health beverage of Embodiment 5, and FIG. 9H shows individuals supplied with the health beverage of Embodiment 6.

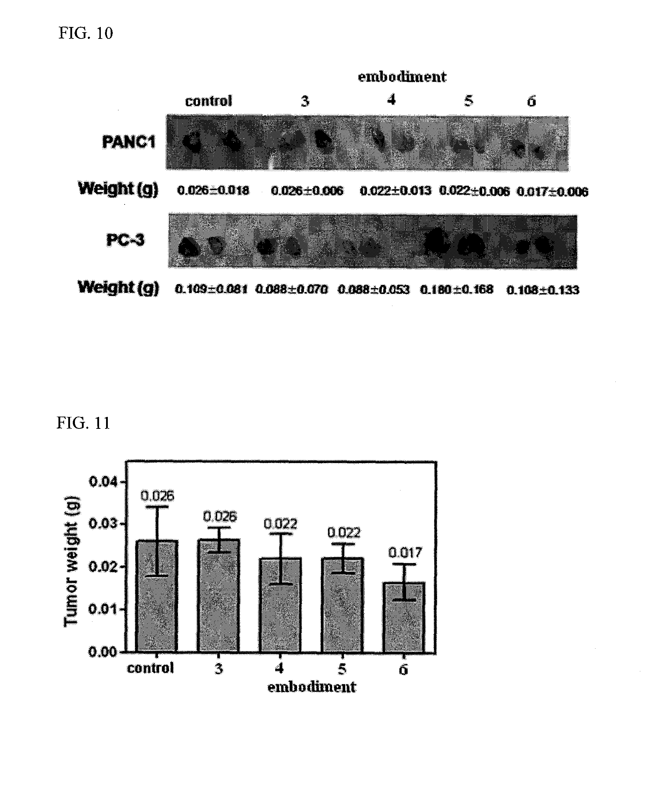

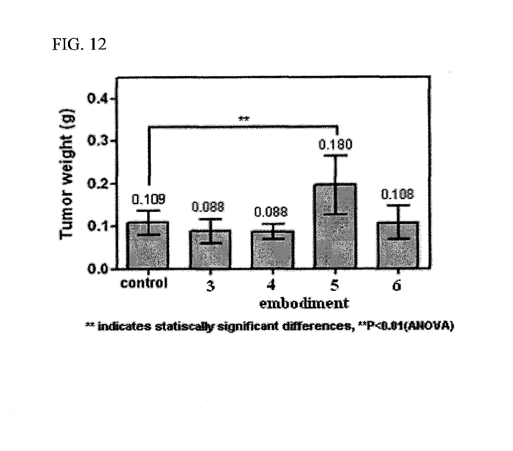

In order to find the effects of the composition of the embodiment on the suppressing of the tumor growth, weights of tumor tissues from the sacrificed mice were measured. The results are shown in FIGS. 10 and 12.

The pancreatic carcinoma occurred in 75% of all individuals to which the cancer cells are inoculated after 15 days have passed. The prostate cancer occurred in 96.7% of all individuals to which the cancer cells are inoculated after 15 days have passed. However, the hepatocellular carcinoma occurred in only 0.1 % of the all individuals. This may result from the low viability of HepG2 cells that are the hepatocellular carcinoma cell lines.

As shown in FIGS. 10 and 11, the mean weight of the pancreatic carcinoma of the experimental groups having the health beverages of Embodiments 3-6 were less than the control group. When having the beverage of Embodiment 6, the weight of the pancreatic carcinoma was greatly reduced. That is, as the concentration of the composition of the embodiment increases, the anti-cancer effect is further enhanced.

In can be noted from FIGS. 10 and 12, when having the health beverages of Embodiments 3, 4, and 6, the weight of the prostate cancer is less than the control group. However, when having the health beverage of Embodiment 5, the weight of the tumor was further increased than the control group.

Accordingly, the composition of the embodiment is more effective for the pancreatic carcinoma than the prostate cancer under the same concentration.

<Test Example 6> Effect on Generation of p53 and p21 Proteins

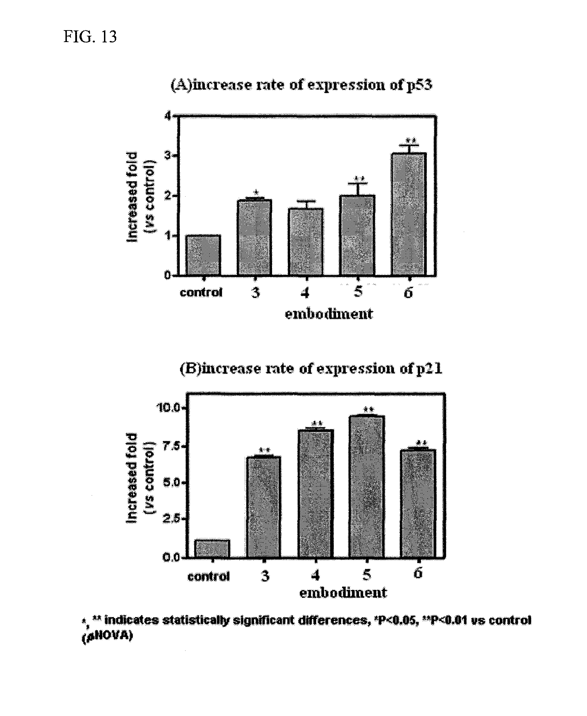

After drinkable water containing the health beverages of Embodiments 3-6 were fed to nude mice to which the cancer cells are inoculated in the same method as Test Example 5 for 2 weeks, variation of generation of p53 and p21 proteins in pancreatic carcinoma tissue extracted from the mice was observed using the western blot method. The results are shown in FIG. 13.

FIG. 13 shows comparison of increasing rates when processed with the beverages of Embodiments 3-6 with reference to generation of p53 and p21 (1.0) of the control group which is not processed with the beverages of Embodiments 3-6. As shown in FIG. 13, when processed with the health beverages of Embodiments 3-6, the generation of the p53 and p21 proteins was greatly increased as compared with the control group. Particularly, for the experimental groups having the health beverages of Embodiments 5 and 6, the generation of the p53 protein was increased 2.0 and 3.0 times as compared with the control group and the generation of the p21 protein that is the subordinate gene of the p53 was increased 6 times as compared with the control group.

This shows that the pharmaceutical composition of the embodiment increases the generation of the anti-cancer proteins and thus suppresses the growth of the cancer cells.

As described above, the pharmaceutical composition of the embodiment increases the generation of the anti-cancer proteins and thus suppresses the growth of the cancer cells. The composition is effective in suppressing the pancreatic carcinoma, hepatocelluar carcinoma, prostate cancer, and the like. Particularly, the composition is effective in suppressing the pancreatic carcinoma even with low concentration.

According to the embodiments, since the pharmaceutical composition is formed of naturally-occurring materials and thus it does not affect the normal cells while suppressing the proliferation of the cancer cells. Therefore, side effects are insignificant even when patients take large doses of the composition over a long time as compared with related art anti-cancer agents. In addition, the composition can be constituted as a health food. Therefore, when the composition can be provided in the form of teas and beverages, the sense of taste is improved allowing easy consumption by patients.

In addition, since the composition increases the generation of the cancer suppressor gene and thus suppresses the growth of the cancer cells, the cancer can be effectively treated and prevented. Particularly, although surgical, chemical and radiation treatment methods are applied for treating pancreatic carcinoma, no other effective treatment has been developed yet. Therefore, it is expected that the pharmaceutical composition of the embodiments can be used to treat pancreatic carcinoma.

Furthermore, the pharmaceutical composition of the embodiments is further effective in enhancing the liver function and is cheaper than the related art drinks containing naturally-occurring medicines. Therefore, the composition works to commercializing advantage.

Exemplary embodiments have been disclosed herein, and although specific terms are employed, they are used and are to be interpreted in a generic and descriptive sense only and not for purpose of limitation. Accordingly, it will be understood by those of ordinary skill in the art that various changes in form and details may be made without departing from the spirit and scope of the present invention as set forth in the following claims.

## Claims

1. A composition for treating and preventing cancer, comprising *rhodiola sachalinensis* and *oldenlandia diffusa.*

2. The composition of claim 1, wherein *the rhodiola sachalinensis* is 10-70 parts by weight for the composition of 100 parts by weight.

3. The composition of claim 1 or claim 2, wherein the *oldenlandia diffusa* is 20-80 parts by weight for the composition of 100 parts by weight.

4. The composition of at least one of claims 1 to 3, further comprising *cistanche deserticola.*

5. The composition of at least one of claims 1 to 4, wherein the *cistanche deserticola* is 10-40 parts by weight for the composition of 100 parts by weight.

6. The composition of at least one of claims 1 to 5, further comprising one selected from the group consisting of *Torilis japonica, Cuscuta japonica Chois, Polygala tenuifolia,* and a mixture thereof.

7. The composition of claim 6, wherein the *Torilis japonica, Cuscuta japonica Chois* or *Polygala tenuifolia* is 5-15 parts by weight for the composition of 100 parts by weight.

8. The composition of at least one of claims 1 to 7, wherein the composition increases expression of a cancer suppressor gene.

9. The composition of at least one of claims 1 to 8, wherein the cancer is one of pancreatic cancer, liver cancer, stomach cancer, colon cancer, uterine cancer, breast cancer, lung cancer, and prostrate cancer.

10. The composition of at least one of claims 1 to 9, the composition is provided in the form of an extract or powder.

11. A health food for ameliorating and preventing cancer, comprising the composition of any one of claims 1 to 10 as an effective ingredient.

12. The health food of claim 11, wherein the health food is a health beverage.

13. The health food of claim 11, wherein the health food is a natural tea.

14. The health food of at least one of claims 11 to 13, wherein an extract of the composition is 0.001 to 30 parts by weight for the health food of 100 parts by weight.

15. Use of a composition according to at least one of claims 1 to 10 for the manufacture of a medicament for the treatment or prophylaxis of cancer.
